# EUROPEAN PATENT SPECIFICATION

(11) **EP 4 278 227 B1**
(45) Date of publication and mention of the grant of the patent: **08.10.2025**
(21) Application number: 22700481.9
(22) Date of filing: 12.01.2022
(51) Int. Cl.: G02B 21/16, G02B 21/36, G02B 27/10, G02B 27/14, G01J 3/00

(54) **MICROSCOPE DEVICE**
MIKROSKOPVORRICHTUNG
DISPOSITIF DE MICROSCOPE

(30) Priority: 12.01.2021 DE 102021100350
(43) Date of publication of application: 22.11.2023
(73) Proprietor: Miltenyi Biotec B.V. & Co. KG, 51429 Bergisch Gladbach (DE)
(72) Inventor: UHL, Rainer, 51429 Bergisch Gladbach (DE)
(86) International application number: PCT/EP2022/050491
(87) International publication number: WO 2022/152720

(56) References cited:
- US-A1- 2009 309 049
- SOBOLEV KIRILL ET AL: "Advanced optical design for DNA sequencing systems", CURRENT DEVELOPMENTS IN LENS DESIGN AND OPTICAL ENGINEERING XII; AND ADVANCES IN THIN FILM COATINGS VII, SPIE, 1000 20TH ST. BELLINGHAM WA 98225-6705 USA, vol. 8128, no. 1, 8 September 2011 (2011-09-08), pages 1 - 11, XP060019786, DOI: 10.1117/12.894268
- HAGA TAKANOBU ET AL: "Simultaneous four-color imaging of single molecule fluorophores using dichroic mirrors and four charge-coupled devices", REVIEW OF SCIENTIFIC INSTRUMENTS, AMERICAN INSTITUTE OF PHYSICS, 2 HUNTINGTON QUADRANGLE, MELVILLE, NY 11747, vol. 82, no. 2, 4 February 2011 (2011-02-04), pages 23701 - 23701, XP012146158, ISSN: 0034-6748, DOI: 10.1063/1.3524570
- ANONYMOUS: "ORCA-D2 Dual CCD Camera - Product Brochure", 5 March 2012 (2012-03-05), XP055554401, Retrieved from the Internet <URL:https://www.biovis.com/images/cameras/e_orcad2.pdf> [retrieved on 20190211]

## Description

### BACKGROUND

The invention relates to a camera-based microscope which employs four-color distinction capabilities to provide a maximally contrast-rich fluorescence respectively transmitted light image.

Typically, camera detectors used in microscopy are monochrome. A multicolor microscope is disclosed in WO 2020/038752, which relates to a microscope device having dual emission detection capabilities. It employs two cameras and places a dichroic beam splitter into the finite optical space between the microscope and the two cameras, which record the two desired spectral regions. In order not to distort the transmitted spectral image, the dichroic is kept as thin and the reflection angle as small as possible. However, since a thin substrate tends to compromise flatness and hence the quality of the reflected image, an optimal thickness always reflects a compromise between the quality of the transmitted and the reflected image.

A similar technology is disclosed in US 2018/0067327 wherein an image beam is divided by a dichroitic beam splitter into two desired spectral regions which are then guided onto one camera.

SOBOLEV KIRILL ET AL: "Advanced optical design for DNA sequencing systems", CURRENT DEVELOPMENTS IN LENS DESIGN AND OPTICAL ENGINEERING XII; AND ADVANCES IN THIN FILM COATINGS VII, SPIE, 1000 20TH ST. BELLINGHAM WA 98225-6705 USA, vol. 8128, no. 1, 8 September 2011 (2011-09-08), pages 1-11, DOI: 10.1117/12.894268, discloses a microscope device for DNA sequencing comprising three dichroic splitters and four CCD cameras for imaging four spectral bands.

HAGA TAKANOBU ET AL: "Simultaneous four-color imaging of single molecule fluorophores using dichroic mirrors and four charge-coupled devices", REVIEW OF SCIENTIFIC INSTRUMENTS, AMERICAN INSTITUTE OF PHYSICS, 2 HUNTINGTON QUADRANGLE, MELVILLE, NY 11747, vol. 82, no. 2, 4 February 2011 (2011-02-04), pages 23701-23701, ISSN: 0034-6748, DOI: 10.1063/1.3524570, discloses a total-internal-reflection (TIR) microscope comprising three dichroic mirrors and four CCD cameras for simultaneous four-color imaging.

US 2009/309049 A1 discloses a microscope device wherein a sample beam is divided with a dichroic beam splitter into two beams which are guided via two reflection elements and the dichroic beam splitter to the detector of one camera.

The product brochure of the "ORCA-D2 Dual CCD Camera", 5 March 2012 (2012-03-05), https:// www.biovis.com/images/cameras/e_orcad2.pdf, discloses a microscope device wherein a sample beam is divided with a dichroic beam splitter into two beams which are guided via three reflection elements to the detector of one camera.

### SUMMARY

It is an object of the invention to provide for a microscope device capable of separating four spectral regions with two cameras only.

Object of the invention is therefore a microscope device according to claim 1.

Such microscope devices are especially useful for detecting multiple spectral ranges emitted by a sample which is often the case in sequencing DNA/RNA molecules. To avoid damaging of the sample, the interaction between the light and the sample should be kept as short as possible. Since the device of the invention can detect at least 4 different spectral ranges simultaneously, the use of the microscope device as disclosed herein in a sequencing-by-synthesis process it is a further object of the invention.

### BRIEF DESCRIPTION OF THE DRAWINGS

Fig. 1 and 2 show two embodiments of the path of light of the microscope of the invention

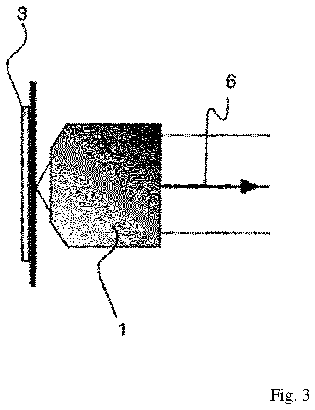

Fig. 3 shows a part of the device upstream of sample beam (6)

### DETAILED DESCRIPTION

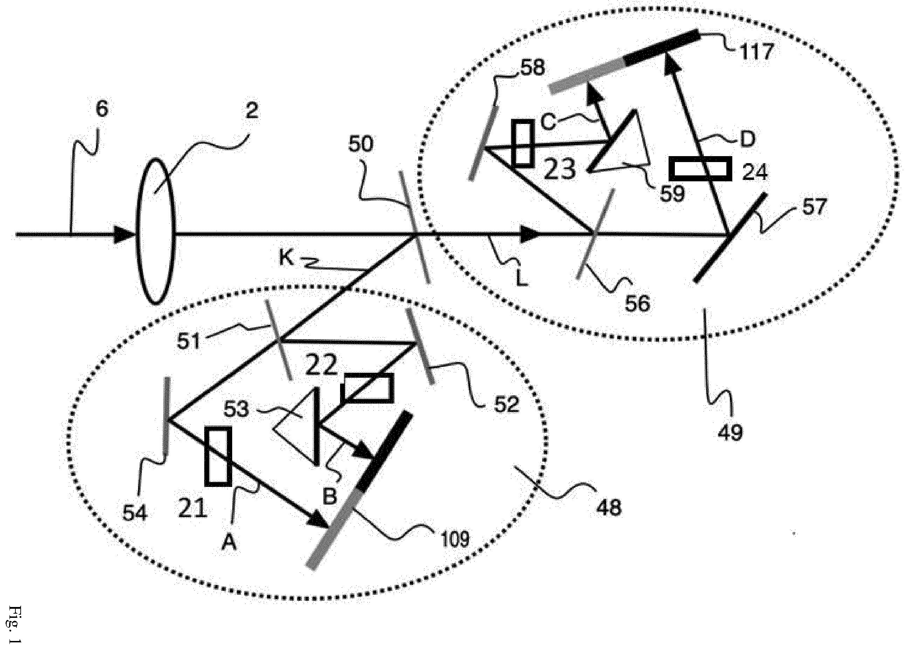

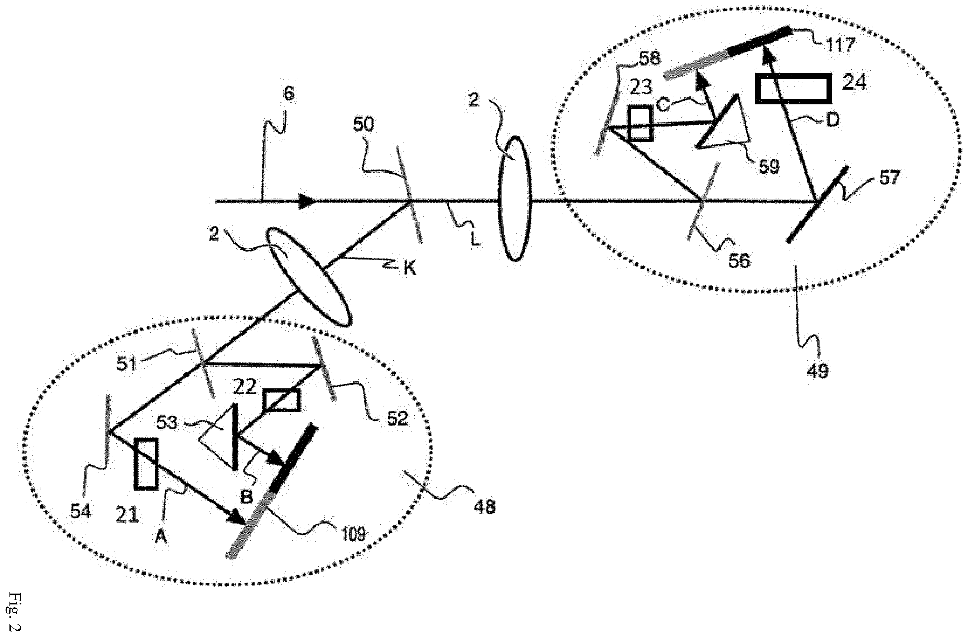

In the device of the invention as shown in Fig. 1 or 2, light originating from the sample (either transmitted or emitted) is separated into four spectral regions by three dichroic beam splitters, and the resulting image beams A, B, C and D are directed to two cameras (109, 117), whereby each of the two cameras records two spectral regions side-by-side on its sensor-chip. This is facilitated by the fact that high-end camera chips are available for image fields much bigger (for example 36 x 24 mm) than the image field of current microscopes, which maximally covers a square field of 17 x 17 mm. Alternatively, TDI cameras are used.

The microscope according to the invention may comprise a at least one camera (109, 117) which is of charge-coupled device (CCD) type, electron-multiplying charge-coupled device (EM-CCD) type, complementary metal-oxide-semiconductor (CMOS) type, scientific complementary metal-oxide-semiconductor (CMOS) type, time delayed integration (TDI) type or a combination thereof.

The microscope is equipped to detect four spectral ranges, for example 405, 488, 561 and 638 nm or 375 nm, 473 nm, 532 nm, und 660 nm. To this end, preferably the cut-on wavelengths are chosen to 488, 561 and 638 nm as to separate the emission into the four spectral regions between the excitation wavelengths. Preferable, the respective spectral ranges of light of the first, second, third and fourth beams (interchangeable with terms A, B, C, D) are between 473 nm and 532 nm; 532 nm and 594 nm, 594 nm and 660 nm; 633 nm and 660 nm. It should be noted that these spectral ranges of light are given by way of example and depend on the function of the beam splitters, i.e. are not necessary bounded to this sequence.

In a preferred embodiment, at least one of the dichroic beam splitters (50, 51, 56) is arranged (tiled) in the path of light as to minimize or avoid the chromatic error of the light detected by the camaras. Since the chromatic error depends on the spectral range / the wavelength of the light, the angular position of the dichroic beam splitters (50, 51, 56) may be same or different.

Accordingly, the tilt angles of first, second and third dichroic beam splitter (50, 51, 56) with the respective residual images may be independently between + 45° and - 45°, preferable independently between + 30° and - 30° or independently between + 25° and - 25°. or independently between + 15° and - 15°. In any case, the tilt angles of the first and third dichroic beam splitters (50, 56) with the respective residual beams may be in opposing directions.

The light sources preferable provide light having a spectral range of wavelengths of 300 to 1750 nm, preferable 300 - 800 nm like white light, laser light, or LED light. The sample may be subjected to the light "as is" or may be provided with fluorescence or phosphorenscence agent to mark regions or interest. To avoid damaging the sample, preferable light sources producing light with longer wavelengths are used, such as 525 and 635 nm.

Accordingly, the sample beam may be or comprise fluorescence or phosphorenscence radiation originating from the sample (3) or radiation transmitted or reflected by the sample (3).

Further, the microscope device may be provided with at least one focusing element (2) into the beam-path of the sample beam upstream of the first beam splitter (shown in Fig. 1). In alternative, two or more focusing elements (2) may be provided in the sample beam downstream of the first beam splitter as shown in Fig. 2.

The focusing elements may consist or comprise at one lens or at least one objective or a combination thereof.

In another embodiment, the microscope device according to the invention may be provided with one or more optical element (21, 22, 23, 24) into the beam-path of first, second, third and/or fourth image beams A, B, C, D. Such optical elements are capable of focal plane or image plane shift and can optionally be inserted and removed from the beam-paths with an appropriate device. Suitable optical elements have a higher refractive index than the surrounding medium and may consist of coated or uncoated glass or polymer. Further, the optical elements can be provided with a filter for chromatic correction of any optical distortion which caused by the dichromatic beam splitters

Fig. 1 and 2 show a part of the device of the invention, which uses two identical optical arrangements (48) and (49) for each of the two camera-chips (109) and (117). A beam splitter (50), placed in the finite optical space between tube-lens (2) and the camera-chips (9) respectively (18), splits the image beam (6) into two beams (K = A + B) and (L = C + D). In the optical arrangement (48), beam splitter (51) splits K into beams A and B, whereas in optical arrangement (49) a beam splitter (56) splits L into beams C and D. The reflected beam (B) needs two more reflections at mirrors (52) and (53), whereas the transmitted beam (A) needs only one reflection at surface (54), before it reaches the detector (109). (L), the beam transmitted by dichroic (50) is split into (C) by being reflected at dichroic (56), and into (D), which is transmitted at dichroic (56). As in (48), the transmitted fraction (D), requires a single reflection at a reflecting element (57), whereas the reflected fraction (C) requires two more reflections at reflecting surfaces (58) and (59).

All beams reflected by a dichroic beam splitter may carry ghost-image information. In Fig. 1 and 2, this applies to spectral regions (A), (B) and (C), which need suitable band-pass filters in front of the detectors, or to overcome spatial restraints, suitable filter characteristics for reflecting surfaces (54), (52) respectively (53) and (58) respectively (59).

In order to minimize (mostly spherical, astigmatic, or comatic) aberrations, the tilt-angle of the two dichroic elements (50) and (51) is kept at about 25° and at opposing angles, as to compensate for chromatic aberrations.

The thickness of the dichroic beam splitters is kept as small as possible (usually 1-3 mm) as to minimize thickness-related aberrations in transmission, but thick enough to maintain flatness of the reflecting surfaces, which is of paramount importance for image quality of the reflected fraction of the beam. (Fig. 1). Alternatively, in order to minimize (mostly spherical, astigmatic, or comatic) aberrations, one dichroic element is positioned between the objective lens (1) and the focusing element (2), allowing for larger tilt angles (preferably 45°) at this dichroic position and allowing for larger thickness (usually 3 mm, possibly 1 mm to 5 mm).

All three spectral regions (A), (B) and (C), which have undergone reflections at dichroic elements, may carry ghost-images, resulting from reflections on the rear (exit) side of the respective dichroic beam splitters (50, 51 and 56). While these ghost-images usually contain less than 1% of the transmitted image information, this may still lead to significant image degradation in cases where the reflected signal is weak and the sum of the transmitted signals is large. The cure for this is to bring appropriate bandpass filters or optical elements (21, 22, 23 and 24) into the beam-path.

Further, the reflection element (52) and/or (53) may be provided with a filter layer having the same optical properties as first dichroic beam splitter (50) and/or as second dichroic beam splitter (51). The reflection element (54) may be provided with a filter layer having the same optical properties as first dichroic beam splitter (50). The reflection element (58) and/or (59) may be provided with a filter layer having the same optical properties as third dichroic beam splitter (56).

Further, reflection element (52) and/or reflection element (53) may be provided with a filter layer having the same optical properties as second dichroic beam splitter (51).

In addition, the microscope device of the invention may be provided with at least one focusing element (2) into the beam-path of the sample beam in order to create an image (6). In alternative (as shown in Fig. 2), the least one focusing element (2) may be provided into the beam-path of the image L and/or K. The focusing element (2) may consist or comprise at one lens or at least one objective or a combination thereof.

In case the respective dichroic beam splitters are long-pass filters, a short-pass filter can replace the bandpass, if the dichroic beam splitter is a short-pass, one needs long-pass filter.

The microscope device according to the present invention allows to differentiate the image of a color-labelled object with respect to up to four spectral regions, both in fluorescence-emission and in transmitted light absorption. Preferable. the optical path-lengths are identical for all spectral ranges (color-channels) and all images lie in the plane of the respective detector chip (camera).

This holds for an optimally corrected optical system. In the real world, the optical layout may be used to correct for longitudinal color-imperfections by adjusting the optical path-lengths accordingly.

In an unstained sample or for a transmitted or reflected light image, however, a volitional detuning of the path-lengths may be used to look at two or more focal depths simultaneously and to reconstruct contrast-enriched images from images taken at different focal positions. For example, a dichroic ensemble, designed for separating the emission excited by a 405 and a 488 nm laser, divides the light of a white light emitting diode into two spectral regions below and above 488 nm.

By suppressing ghost-images in the longer wavelength-channel >488 nm by inserting a optical element (22) into the beam-path (11), and by providing means to remove the optical element (22) from the beam, the thickness of the optical element (22) determines the path-length difference of beams A and B. The same arrangement can be provided with optical element (21), (23) and/or (24).

With a 40x objective, removing a filter-substrate of thickness 2 mm, provides a focal displacement the two images recorded by camera chip (109) of 416 nm. Obviously other means for providing a suitable path-length difference between two or more color channels are also possible according to the invention.

### USE OF DEVICE

The microscope devices of the invention are especially useful in methods for detecting multiple spectral ranges which are emitted during sequencing of DNA/RNA molecules, in particular for sequencing-by-synthesis processes to obtain DNA or RNA sequence information of a biological sample.

Preferable, the sequencing-by-synthesis process is performed by hybridization of nucleotides provided with different dyes to the DNA or RNA of the biological sample and wherein the dyes emit light upon excitation by the one or more light sources in the spectral ranges A, B, C and D or combinations thereof. Such sequencing-by-synthesis process and the required dyes are known to the person skilled in the art

## Claims

1. A microscope device comprising:
a microscope objective (1);
one or more light sources configured to generate light;
at least a first (50), a second (51) and a third dichroic beam splitter (56),
a first camera (109) comprising a detector and a second camera (117) comprising a detector,
wherein the light generated by the one or more light sources is configured to interact with a sample (3) to thereby produce a sample beam (6),
wherein the microscope device is configured to
- divide the sample beam (6) with the first dichroic beam splitter (50) into a first sample beam (K) and a second sample beam (L) wherein the first sample beam (K) and the second sample beam (L) have different spectral ranges of light,
- divide the first sample beam (K) with the second dichroic beam splitter (51) into a first beam (A) having a first spectral range of light and a second beam (B) having a second spectral range of light,
- divide the second sample beam (L) with the third dichroic beam splitter (56) into a third beam (C) having a third spectral range of light and a fourth beam (D) having a fourth spectral range of light,
**characterised in that**
the microscope device additionally comprises a first (54), second (52), third (53), fourth (58), fifth (59) and sixth reflection element (57),
the microscope device is configured to guide the first beam (A) via the first reflection element (54) on the detector of the first camera (109) and to guide the second beam (B) via the second (52) and third reflection element (53) on the detector of the first camera (109), and
the microscope device is configured to guide the third beam (C) via the fourth (58) and fifth reflection element (59) on the detector of the second camera (117) and to guide the fourth beam (D) via the sixth reflection element (57) on the detector of the second camera (117).

2. Microscope device according to claim 1 **characterized in that** the tilt angles of the first, second and third dichroic beam splitter (50, 51, 56) with the respective beams are independently between + 45° and - 45°.

3. Microscope device according to claim 1 or 2 **characterized in that** the first reflection element (54) is provided with a filter layer having the same optical properties as the first dichroic bearr splitter (50).

4. Microscope device according to any of the claims 1 to 3 **characterized in that** the fourth reflection element (58) and/or the fifth reflection element (59) is provided with a filter layer having the same optical properties as third dichroic beam splitter (56).

5. Microscope device according to any of the claims 1 to 4 **characterized in that** the second reflection element (52) and/or the third reflection element (53) is provided with a filter layer having the same optical properties as first dichroic beam splitter (50).

6. Microscope device according to any of the claims 1 to 5 **characterized in that** the second reflection element (52) and/or the third reflection element (53) is provided with a filter layer having the same optical properties as second dichroic beam splitter (51).

7. Microscope device according to any of the claims 1 to 6 **characterized in that** the one or more light sources are configured to provide light having a spectral range of 300 to 1750 nm.

8. Microscope device according to any of the claims 1 to 7 **characterized in** additionally comprising at least one focusing element (2) in the beam-path of the sample beam (6).

9. Microscope device according to claim 8 **characterized in that** the at least one focusing element (2) comprises at least one lens or at least one objective or a combination thereof.

10. Microscope device according to any of the claims 1 to 9 **characterized in** additionally comprising one or more optical element (21, 22, 23, 24) in the beam-path of the first (A), second (B), third (C) and/or fourth beam (D).

11. Microscope device according to claim 10 **characterized in that** the one or more optical element (21, 22, 23, 24) are capable of being inserted and removed from the beam-paths.

12. Microscope device according to any of the claims 1 to 11 **characterized in that** the sample beam (6) comprises fluorescence or phosphorenscence radiation or transmitted light or reflected light originating from the sample (3).

13. Microscope device according to any of the claims 1 to 12 **characterized in that** the first (A) and second (B) beam and/or the third (C) and fourth beam (D) originate from different positions on the sample (3); wherein the beams of the different positions can be brought in alignment by moving the sample (3) relative to the cameras.

14. Use of the Microscope device according to any of the claims 1 to 13 in a sequencing-by-synthesis process.

15. Use according to claim 14 **characterized in that** the sequencing-by-synthesis process is performed by hybridization of nucleotides provided with different dyes to the DNA or RNA of the biological sample and wherein the dyes emit light upon excitation by the one or more light sources in the first, second, third and fourth spectral range or a combination thereof.

## Patentansprüche

1. Mikroskopvorrichtung, umfassend:
ein Mikroskopobjektiv (1);
eine oder mehrere Lichtquellen, die so konfiguriert sind, dass sie Licht erzeugen;
mindestens einen ersten (50), einen zweiten (51) und einen dritten dichroitischen Strahlteiler (56),
eine erste Kamera (109), umfassend einen Detektor und eine zweite Kamera (117), umfassend einen Detektor,
wobei das von der einen oder den mehreren Lichtquellen erzeugte Licht so konfiguriert ist, dass es mit einer Probe (3) interagiert, um dadurch einen Probenstrahl (6) zu erzeugen,
wobei die Mikroskopvorrichtung konfiguriert ist zum
- Aufteilen des Probenstrahls (6) mit dem ersten dichroitischen Strahlteiler (50) in einen ersten Probenstrahl (K) und einen zweiten Probenstrahl (L), wobei der erste Probenstrahl (K) und der zweite Probenstrahl (L) unterschiedliche Spektralbereiche des Lichts aufweisen,
- Aufteilen des ersten Probenstrahls (K) mit dem zweiten dichroitischen Strahlteiler (51) in einen ersten Strahl (A) mit einem ersten Spektralbereich des Lichts und einen zweiten Strahl (B) mit einem zweiten Spektralbereich des Lichts,
- Aufteilen des zweiten Probenstrahls (L) mit dem dritten dichroitischen Strahlteiler (56) in einen dritten Strahl (C) mit einem dritten Spektralbereich des Lichts und einen vierten Strahl (D) mit einem vierten Spektralbereich des Lichts,
**dadurch gekennzeichnet, dass**
die Mikroskopvorrichtung zusätzlich ein erstes (54), zweites (52), drittes (53), viertes (58), fünftes (59) und sechstes Reflexionselement (57) umfasst,
die Mikroskopvorrichtung konfiguriert ist, um den ersten Strahl (A) über das erste Reflexionselement (54) auf den Detektor der ersten Kamera (109) zu lenken und den zweiten Strahl (B) über das zweite (52) und dritte Reflexionselement (53) auf den Detektor der ersten Kamera (109) zu lenken, und
die Mikroskopvorrichtung konfiguriert ist, um den dritten Strahl (C) über das vierte (58) und fünfte Reflexionselement (59) auf den Detektor der zweiten Kamera (117) zu lenken und den vierten Strahl (D) über das sechste Reflexionselement (57) auf den Detektor der zweiten Kamera (117) zu lenken.

2. Mikroskopvorrichtung nach Anspruch 1, **dadurch gekennzeichnet, dass** die Neigungswinkel des ersten, zweiten und dritten dichroitischen Strahlteilers (50, 51, 56) mit den jeweiligen Strahlen unabhängig voneinander zwischen +45° und -45° liegen.

3. Mikroskopvorrichtung nach Anspruch 1 oder 2, **dadurch gekennzeichnet, dass** das erste Reflexionselement (54) mit einer Filterschicht versehen ist, die die gleichen optischen Eigenschaften wie der erste dichroitische Strahlteiler (50) aufweist.

4. Mikroskopvorrichtung nach einem der Ansprüche 1 bis 3, **dadurch gekennzeichnet, dass** das vierte Reflexionselement (58) und/oder das fünfte Reflexionselement (59) mit einer Filterschicht versehen ist, die die gleichen optischen Eigenschaften wie der dritte dichroitische Strahlteiler (56) aufweist.

5. Mikroskopvorrichtung nach einem der Ansprüche 1 bis 4, **dadurch gekennzeichnet, dass** das zweite Reflexionselement (52) und/oder das dritte Reflexionselement (53) mit einer Filterschicht versehen ist, die die gleichen optischen Eigenschaften wie der erste dichroitische Strahlteiler (50) aufweist.

6. Mikroskopvorrichtung nach einem der Ansprüche 1 bis 5, **dadurch gekennzeichnet, dass** das zweite Reflexionselement (52) und/oder das dritte Reflexionselement (53) mit einer Filterschicht versehen ist, die die gleichen optischen Eigenschaften wie der zweite dichroitische Strahlteiler (51) aufweist.

7. Mikroskopvorrichtung nach einem der Ansprüche 1 bis 6, **dadurch gekennzeichnet, dass** die eine oder die mehreren Lichtquellen so konfiguriert sind, dass sie Licht in einem Spektralbereich von 300 bis 1.750 nm bereitstellen.

8. Mikroskopvorrichtung nach einem der Ansprüche 1 bis 7, **dadurch gekennzeichnet, dass** sie zusätzlich mindestens ein Fokussierelement (2) im Strahlengang des Probenstrahls (6) aufweist.

9. Mikroskopvorrichtung nach Anspruch 8, **dadurch gekennzeichnet, dass** das mindestens eine Fokussierelement (2) mindestens eine Linse oder mindestens ein Objektiv oder eine Kombination davon umfasst.

10. Mikroskopvorrichtung nach einem der Ansprüche 1 bis 9, **dadurch gekennzeichnet, dass** sie zusätzlich ein oder mehrere optische Elemente (21, 22, 23, 24) im Strahlengang des ersten (A), zweiten (B), dritten (C) und/oder vierten Strahls (D) aufweist.

11. Mikroskopvorrichtung nach Anspruch 10, **dadurch gekennzeichnet, dass** das eine oder die mehreren optischen Elemente (21, 22, 23, 24) in die Strahlengänge eingefügt und aus diesen entfernt werden können.

12. Mikroskopvorrichtung nach einem der Ansprüche 1 bis 11, **dadurch gekennzeichnet, dass** der Probenstrahl (6) Fluoreszenz- oder Phosphoreszenzstrahlung oder Durchlicht oder reflektiertes Licht umfasst, das von der Probe (3) ausgeht.

13. Mikroskopvorrichtung nach einem der Ansprüche 1 bis 12, **dadurch gekennzeichnet, dass** der erste (A) und der zweite (B) Strahl und/oder der dritte (C) und der vierte Strahl (D) von unterschiedlichen Positionen auf der Probe (3) ausgehen; wobei die Strahlen der unterschiedlichen Positionen durch Bewegen der Probe (3) relativ zu den Kameras in Ausrichtung gebracht werden können.

14. Verwendung der Mikroskopvorrichtung nach einem der Ansprüche 1 bis 13 in einem Sequencing-by-Synthesis-Verfahren.

15. Verwendung nach Anspruch 14, **dadurch gekennzeichnet, dass** das Sequencing-by-Synthesis-Verfahren durch Hybridisierung von Nukleotiden, die mit verschiedenen Farbstoffen versehen sind, an die DNA oder RNA der biologischen Probe durchgeführt wird und wobei die Farbstoffe bei Anregung durch die eine oder die mehreren Lichtquellen Licht im ersten, zweiten, dritten und vierten Spektralbereich oder einer Kombination davon emittieren.

## Revendications

1. Dispositif de microscope comprenant :
un objectif de microscope (1) :
une ou plusieurs sources de lumière configurées pour produire de la lumière ;
au moins un premier (50), un deuxième (51) et un troisième diviseur de faisceau dichroïque (56),
une première caméra (109) comprenant un détecteur et une seconde caméra (117) comprenant un détecteur,
la lumière produite par l'une ou plusieurs sources de lumière étant configurée pour interagir avec un échantillon (3) pour ainsi produire un faisceau d'échantillon (6),
le dispositif de microscope étant configuré pour
- diviser le faisceau d'échantillon (6) avec le premier diviseur de faisceau dichroïque (50) en un premier faisceau d'échantillon (K) et un second faisceau d'échantillon (L) le premier faisceau d'échantillon (K) et le second faisceau d'échantillon (L) ayant différentes plages de lumière spectrale,
- diviser le premier faisceau d'échantillon (K) avec le deuxième diviseur de faisceau dichroïque (51) en un premier faisceau (A) ayant une première plage de lumière spectrale et un deuxième faisceau de lumière (B) ayant une deuxième plage de lumière spectrale,
- diviser le second faisceau d'échantillon (L) avec le troisième diviseur de faisceau dichroïque (56) en un troisième faisceau (C) ayant une troisième plage de lumière spectrale et un quatrième faisceau (D) ayant une quatrième plage de lumière spectrale,
**caractérisé en ce que**
le dispositif de microscope comprend de plus un premier (54), un deuxième (52), un troisième (53), un quatrième (58), un cinquième (59) et un sixième (57) élément de réflexion,
le dispositif de microscope est configuré pour guider le premier faisceau (A) via le premier élément de réflexion (54) sur le détecteur de la première caméra (109) et pour guider le deuxième faisceau (B) via les deuxième (52) et troisième (53) éléments de réflexion sur le détecteur de la première caméra (109) et
le dispositif de microscope est configuré pour guider le troisième faisceau (C) via le quatrième (58) et le cinquième (59) éléments de réflexion sur le détecteur de la seconde caméra (117) et pour guider le quatrième faisceau (D) via le sixième élément de réflexion (57) sur le détecteur de la seconde caméra (117).

2. Dispositif de microscope selon la revendication 1, **caractérisé en ce que** les angles d'inclinaison des premier, deuxième et troisième diviseurs de faisceau dichroïque (50, 51, 56) avec les faisceaux respectifs sont indépendamment compris entre +45° et -45°.

3. Dispositif de microscope selon la revendication 1 ou 2, **caractérisé en ce que** le premier élément de réflexion (54) est fourni avec une couche de filtre ayant les mêmes propriétés optiques que le premier diviseur de faisceau dichroïque (50).

4. Dispositif de microscope selon l'une quelconque des revendications 1 à 3, **caractérisé en ce que** le quatrième élément de réflexion (58) et/ou le cinquième élément de réflexion (59) sont fournis avec une couche de filtre ayant les mêmes propriétés optiques que le troisième diviseur de faisceau dichroïque (56).

5. Dispositif de microscope selon l'une quelconque des revendications 1 à 4, **caractérisé en ce que** le deuxième élément de réflexion (52) et/ou le troisième élément de réflexion (53) sont fournis avec une couche de filtre ayant les mêmes propriétés optiques que le premier diviseur de faisceau dichroïque (50).

6. Dispositif de microscope selon l'une quelconque des revendications 1 à 5, **caractérisé en ce que** le deuxième élément de réflexion (52) et/ou le troisième élément de réflexion (53) sont fournis avec une couche de filtre ayant les mêmes propriétés optiques que le deuxième diviseur de faisceau dichroïque (51).

7. Dispositif de microscope selon l'une quelconque des revendications 1 à 6, **caractérisé en ce que** les une ou plusieurs sources de lumière sont configurées pour fournir de la lumière ayant une plage spectrale de 300 à 1750 nm.

8. Dispositif de microscope selon l'une quelconque des revendications 1 à 7, **caractérisé en** comprenant de plus au moins un élément de focalisation (2) dans le chemin de faisceau du faisceau d'échantillon (6).

9. Dispositif de microscope selon la revendication 8, **caractérisé en ce que** l'au moins un élément de focalisation (2) comprend au moins une lentille ou au moins un objectif ou une de leurs combinaisons.

10. Dispositif de microscope selon l'une quelconque des revendications 1 à 9, **caractérisé en** comprenant de plus un ou plusieurs éléments optiques (21, 22, 23, 24) dans le chemin de faisceau du premier (A), du deuxième (B), du troisième (C) et/ou du quatrième faisceau (D).

11. Dispositif de microscope selon la revendication 10, **caractérisé en ce que** les un ou plusieurs éléments optiques (21, 22, 23, 24) sont capables d'être insérés et enlevés des chemins de faisceau.

12. Dispositif de microscope selon l'une quelconque des revendications 1 à 11, **caractérisé en ce que** le faisceau d'échantillon (6) comprend un rayonnement de fluorescence ou de phosphorescence ou de lumière transmise ou de lumière réfléchie provenant de l'échantillon (3).

13. Dispositif de microscope selon l'une quelconque des revendications 1 à 12, **caractérisé en ce que** le premier (A) et le deuxième faisceau (B) et/ou le troisième (C) et le quatrième faisceau (D) proviennent de différentes positions sur l'échantillon (3) ; les faisceaux des différentes positions pouvant être mis en alignement en déplaçant l'échantillon (3) par rapport aux caméras.

14. Utilisation du dispositif de microscope selon l'une quelconque des revendications 1 à 13, dans un processus de séquençage par synthèse.

15. Utilisation selon la revendication 14, **caractérisée en ce que** le processus de séquençage par synthèse est effectué par hybridation de nucléotides fournis avec différents colorants à l'ADN ou l'ARN de l'échantillon biologique et les colorant émettant de la lumière après excitation par l'une ou plusieurs sources de lumière dans la première, deuxième, troisième et quatrième plage spectrale ou une de leurs combinaisons.
